# EUROPEAN PATENT SPECIFICATION

(11) **EP 2 984 469 B1**
(45) Date of publication and mention of the grant of the patent: **29.03.2017**
(21) Application number: 14717057.5
(22) Date of filing: 09.04.2014
(51) Int. Cl.: A61B 46/10

(54) **PROTECTIVE COVERING FOR A HAND-HELD MEDICAL DEVICE**
SCHUTZABDECKUNG FÜR EINE TRAGBARE MEDIZINISCHE VORRICHTUNG
REVÊTEMENT PROTECTEUR POUR DISPOSITIF MÉDICAL PORTATIF

(30) Priority: 09.04.2013 EP 13162924
(43) Date of publication of application: 17.02.2016
(73) Proprietor: Roche Diabetes Care GmbH, 68305 Mannheim (DE); F. Hoffmann-La Roche AG, 4070 Basel (CH)
(72) Inventor: HARTTIG, Herbert, 67434 Neustadt (DE); LIEDTKE, Sebastian, 69117 Heidelberg (DE); MOCK, Matthias, D-65549 Limburg (DE)
(74) Representative: Pfiz, Thomas
(86) International application number: PCT/EP2014/057136
(87) International publication number: WO 2014/166987

(56) References cited:
- WO-A1-2012/116798
- US-A- 5 490 524
- US-A1- 2010 311 090
- US-A1- 2011 089 175
- US-A1- 2013 048 530

## Description

The invention concerns a protective covering for a hand-held medical device that has a measuring port for a disposable analytical test strip to which a sample can be applied comprising a sleeve which preferably has a reception opening and can be mounted on the device to partially or fully enclose the device in a usable protected state.

A similar protective barrier for medical devices is disclosed in WO 2012/116798. In this document, a casing is shown which includes a plurality of perforations wherein the casing is separable along the perforations to remove the medical device after use. The medical device includes a test element receiving port 14 that is aligned with an open passage of the casing, allowing insertion of the test element through casing and into receiving port. With such a configuration, it is intended to avoid or minimize the use of disinfectants in removing contamination while preventing contamination during use of the medical device so it can be re-used at a later time.

Another similar protective barrier for medical devices is disclosed in US 2003/048530. The two-part form of independent claim 1 is based on this document.

On this basis the object of the invention is to further improve the known test protective barriers and medical devices and to achieve an improved efficiency for safe and reliable use of hand-held medical devices in patient proximity.

The combination of features stated in claim 1 is proposed to achieve this object. Advantageous embodiments and further developments of the invention are derived from the dependent claims.
The invention is based on the idea of avoiding contamination of the device specifically in the area of a sample measuring interface. Correspondingly it is proposed according to the invention
- a test strip adapter formed as an integral part of the sleeve to receive a test strip in alignment with the device port,
- wherein the sleeve including the test strip adapter is arranged as a sealing barrier between the test strip and the device port in the protected state; and
- wherein the test strip adapter has an optical window to establish a sealed optical measuring path between the test strip and the device port.

Thus, the covering fulfills multiple functions, namely as a protective sheath and as an adapter replicating an instrument port. Due to these measures, a direct contact of sample fluid with instrument parts can be prevented in every case. This is specifically important where disposable test strips to which body fluid has to be applied are handled by patients eventually in impaired physical conditions, so that an accurate positioning cannot be guaranteed. Such measurements may include the direct application of fresh blood sampled from a body part, e.g. for blood coagulation or glucose measurements. Specifically in a medical environment, the function as a sealed barrier can reduce the spread of infectious material, both when the device is carried into or out of such an environment. In this connection, it is advantageous when the device is fully enclosed in the sleeve preferably using a sealing technology. Alternatively it is also possible that the device is inserted into the sleeve through the reception opening and only partly enclosed therein.

In order to further allow a precise alignment for a test strip that can be inserted into the test strip adapter by a user, it is advantageous when the test strip adapter has holding elements to engage and hold the test strip in a defined position relative to the measuring port.

A further improvement in this direction can be achieved when the test strip adapter can be detachably connected to the medical device via guiding or latching or plug-in elements.

More specifically, the optical window consists of a transparent material, preferably PET, PMMA, PC, PC-ABS, PS, SAN or ASA.

In order to sanitarily enclose the device, it is advantageous when the sleeve has a first closed end, and when the closure provides a second closed end, such that the device is fully protected against the environment.

Another particularly advantageous embodiment provides that the sleeve is construed as a flexible, unshaped or shapeless member which can be fitted to the shape of the device, such that the sleeve is conformed or adopted to a 3-dimensional design of the device. In this context, it is also preferred when the sleeve consists of a flexible foil material which can be elastically adapted to an outer contour of the device.

With regard to design and usability of the device, it is advantageous when the sleeve is transparent at least in an area covering a display or a control unit of the device, so that the usual handling of the device is not affected.

It is also conceivable that the sleeve comprises first and second sleeve parts which can be connected by adherence in an overlapping joint region, such that the device is fully enclosed in the combined sleeve.

In order to simplify application, it is advantageous when the sleeve comprises a sack-like part which can be turned inside-out or everted over test strip adapter when mouted onto the device.

For a further improvement in convenience it is advantageous when the sleeve comprises tear-off parts in a multi-layered arrangement which can be successively detached from the device for sequential use. In this arrangement, it is also conceivable that the device is designed as a disposable and is discarded after consumption of all tear-off parts.

In a further preferred embodiment the sleeve is sterile and forms an aseptic shielding for the device.

The invention also concerns a hand-held medical device comprising a measuring port for a disposable analytical test strip and a protective covering as outlined above.

The invention is further elucidated in the following on the basis of embodiment examples shown schematically in the drawings, where
- Fig. 1: is a perspective, exploded view of a blood glucose meter and a protective covering in an initial state;
- Fig. 2: shows a partly assembled state of the covering on the meter of fig. 1;
- Fig. 3: shows the meter inside the covering in a protected state ready for use of a test strip;
- Fig. 4: is a perspective, exploded view of a second embodiment with a one-piece covering;
- Fig. 5: shows the assembled state of the embodiment of Fig. 4;
- Fig. 6 and 7: is a top view of an exemplary protective covering in the initial and assembled state;
- Fig. 8: is an expanded view of a test strip adapter of the covering of fig. 6 and 7;
- Fig. 9: is a sectional top view of a sleeve including a test strip adapter for optical measurements.

The drawings show an arrangement of a medical device configured as a portable blood glucose meter 10 for processing of disposable analytical test strips 12 and a protective covering 14 comprising a one- or two-part sleeve 16 which can be mounted on the meter 10 and an integrated test strip adapter 18 for inserting a test strip 12 in the assembled state.

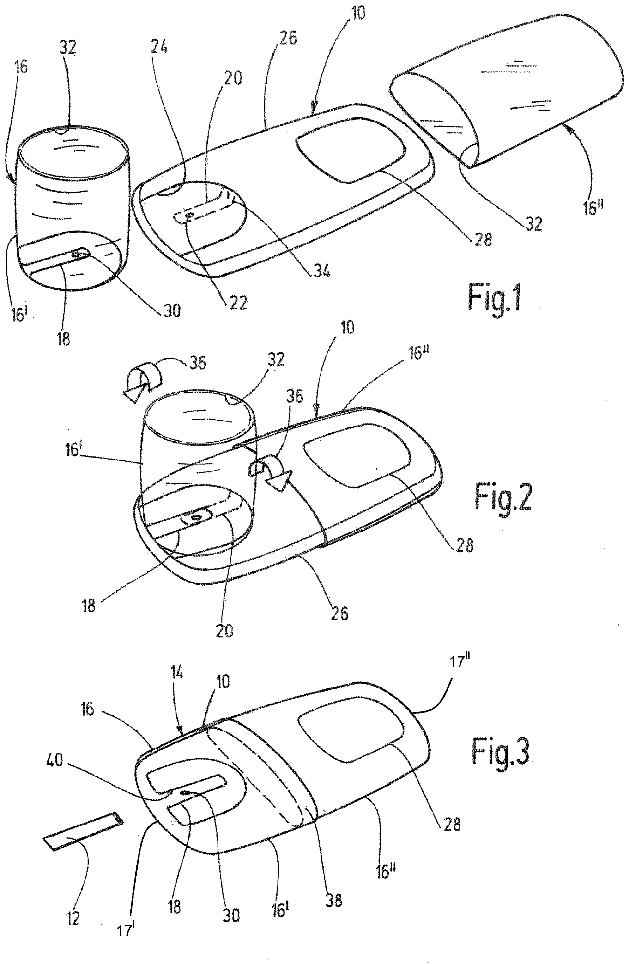

As illustrated in fig. 1 the meter 10 has a measuring port 20 to which the test strip adapter 18 can be aligned. The measuring port 20 is intended for optical measurements on a test strip 12 and comprises a transparent inset 22 closely embedded in a tray 24 of the device housing 26. The transparent inset 22 is arranged in the optical path of a reflectometric measuring device of the meter 10 for a reflectance measurement on a separate test strip 12 loaded with a blood sample, where the result can be obtained on the spot and shown to the user via display 28. Such glucose measurements are known per se to the skilled person and need not to be explained in further detail.

In order to provide a sealing barrier between the measuring port 20 and the test strip 12, the sleeve 16 can be slipped over the device housing 26 and mounted such that an optical window 30 of the test strip adapter 18 is aligned with the transparent inset 22 of the housing 26. For this purpose, both parts 16', 16" of the sleeve 16 have an opening 32 for receiving or being imposed on opposed portions of the elongate housing 26. In order to ensure a tight sealing, the sleeve 16 consists of a flexible foil material which can be elastically adapted to an outer contour of the housing 26 and is preferably transparent at least in an area covering the display 28.

The alignment of the optical window 30 with the transparent inset 22 can be facilitated by means of guiding or latching elements 34 to which the test strip adapter 18 can be detachably connected. In the assembled state, the optical window 30 should closely adhere to the transparent inset 22, such that air gaps are avoided and losses in the optical path are minimised. For further improvement in this regard, the optical window 30 consists of a transparent material selected from the group PET, PMMA, PC, PC-ABS, PS, SAN or ASA.

Fig. 2 further illustrates the assembly of sleeve 16 on the device 10. Opening 32 of the sleeve part 16" is sized to allow the device 10 to be inserted therethrough, so that at least a first half of housing 26 including the display 28 is hermetically sealed. Then, the test strip adapter 18 is connected to the measuring port 20, and sleeve part 16' is slipped over the second half of housing 26. Here, the flexibility of the foil material allows the user to extend and evert the sack-like sleeve part 16' in direction of arrows 36.

Fig. 3 shows the completely mounted state, in which the sleeve parts 16', 16" are connected by adherence in an overlapping joint region 38, such that the device 10 is fully enclosed in the combined sleeve 16. The sleeve parts 16', 16" each have a closed end 17', 17", such that the meter 10 is completely encased. Purposefully, the sleeve 16 including the integrated test strip adapter 18 is sterilized to form an aseptic protective covering 14. In the protected state the test strip 12 can be connected to test strip adapter 18 without direct contact to the meter 10. Test strip adapter 18 includes holding elements 40 to engage and hold the test strip 12 in a defined position relative to the measuring window 30 in alignment with the measuring port 20.

If wanted, the user can tear off and discard the covering 14 as an expendable item. It is also conceivable that the sleeve 16 comprises tear-off parts 16' in a multi-layered arrangement which can be successively detached from the 10 device for sequential use.

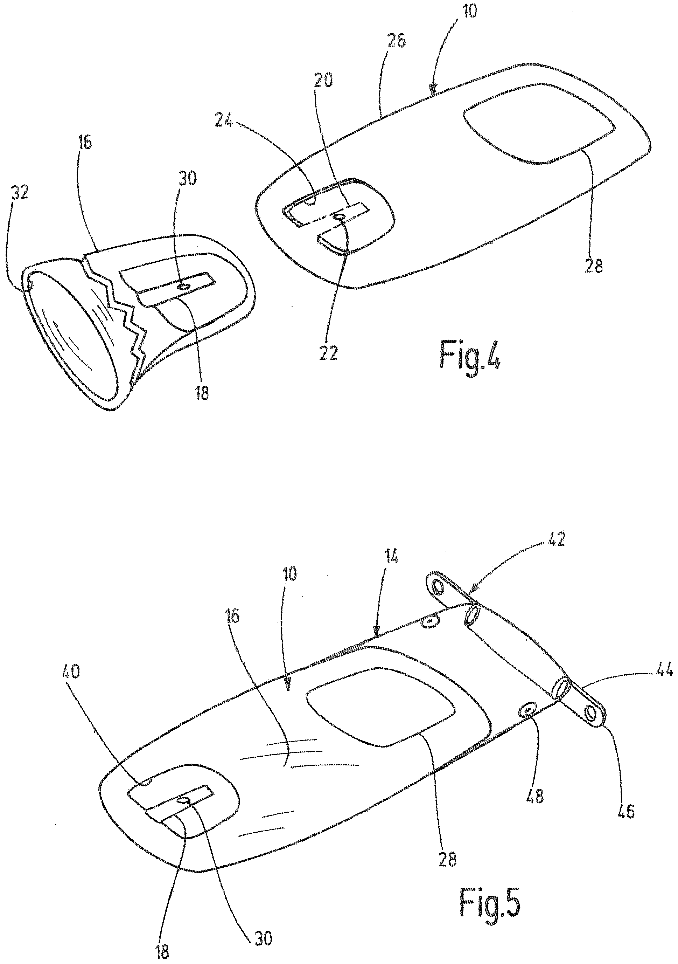

Turning to figs. 4 and 5, a further embodiment of the present invention comprising a one-part sleeve 16 can be seen. Here, similar elements have like reference numerals as previously described. As distinguished to the previous embodiment, the one-part sleeve 16 can be completely slipped over the device 10, such that housing 28 is entirely received. Thereafter, a closure 42 is used to close the reception opening 32 in the overlaying end section of the sleeve 16. The closure 42 is construed as a foil folding fastener 44 which has end flaps 46 that can be snapped on pressure locks 48 on the sleeve 16.

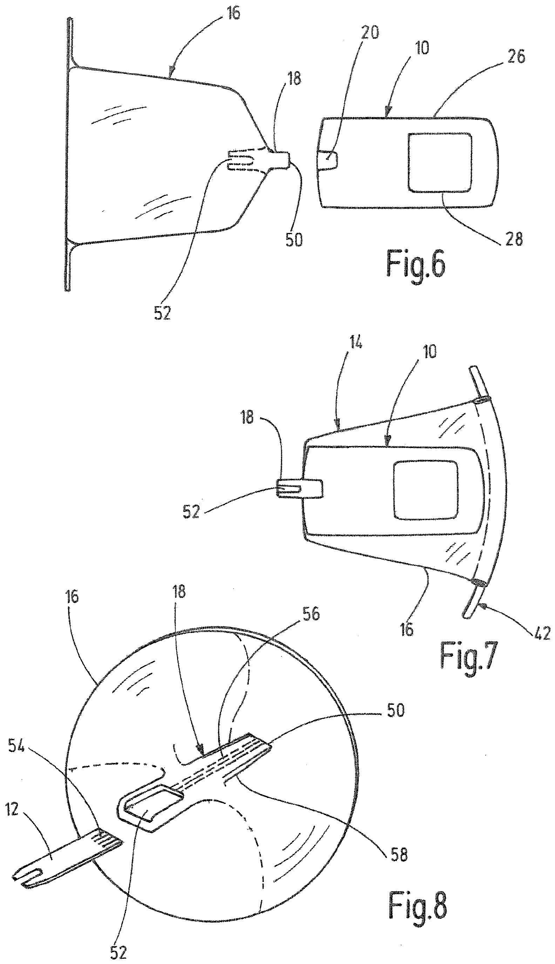

The exemplary device shown in fig. 6 to 8 comprises a glucometer 10 with a measuring port 20 that is intended for electrochemical measurements on a test strip 12. For this purpose, the measuring port 20 is constructed as a female connector which has contact springs for a wired connection to an amperometric measuring device of the meter 10 (not shown). A single-part exemplary sleeve 16 is provided with an integrated electrical test strip adapter 18. As apparent from fig. 7, the exemplary sleeve 16 can be slipped over the device 10 and closed by means of a foil folding fastener 44 as previously described.

As best seen from fig. 8, the exemplary test strip adapter 18 has a distal male connector end 50 which can be plugged into the measuring port 20 and a proximal female connector end 52 which can receive the contact section 54 of test strip 12. The male end 50 is designed similar to the contact section 54, whereas the female end 52 replicates the measuring port 20. In this way, it is also conceivable that the measuring port 20 receives a test strip 12 for a measurement in an unprotected state when the exemplary protective covering 14 is removed.

In the protected state, when the meter 10 is hermetically enclosed in the covering 14, the exemplary test strip adapter 18 forms an electrical connector to provide a galvanic coupling between the test strip 12 and the measuring port 20 of the device 10. Conducting paths 56 are formed within the exemplary test strip adapter 18 from metal, preferably comprising at least partly a noble metal, organic conductors or carbon derivatives, contained in the non-conducting matrix of a carrier 58.

The exemplary test strip adapter 18 may allow an automatic switch-on function of the device 10 when a test strip 12 is inserted.

In case of electrochemical measurements, a corresponding change in resistivity can be detected through the conducting paths 56, and a monitoring circuit of the exemplary device can be operable for a turn-on procedure.

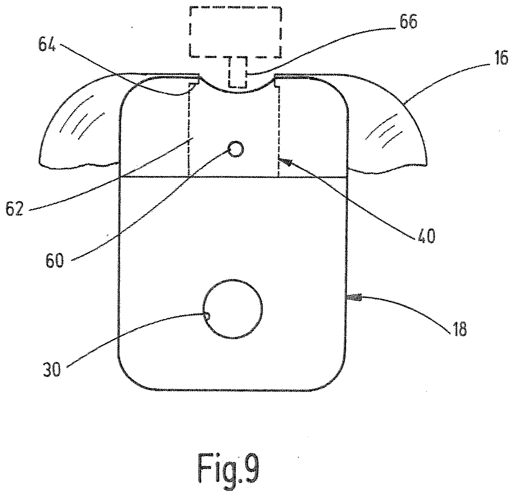

Fig. 9 shows an embodiment of test strip adapter 18 providing a switch-on function for optically working systems. The adapter 18 includes holding elements 40 formed as a pin 60 and a holding-down clamp 62 for a form-locking engagement of an inserted test strip 12. Then, the distal end of the test strip abuts the stop edges and at the same time actuates a microswitch 66 on the side of the device 10 to effectuate power-on.

Correspondingly, an additional aspect of the invention is contemplated as follows: A protective covering 14 comprising a sleeve 16 which can be slipped over a device 10 and includes a test strip adapter 18, wherein the test strip adapter 18 is operable to provide a switch-on function of the device 10 when a test strip 12 is inserted into the test strip adapter 18.

## Claims

1. Protective covering for a hand-held medical device (10) that has a measuring port (20) for a disposable analytical test strip (12) to which a sample can be applied comprising
- a sleeve (16) which preferably has a reception opening (32) and can be mounted on the device (10) to partially or fully enclose the device (10) in a usable protected state,
- a test strip adapter (18) formed as an integral part of the sleeve (16) to receive a test strip (12) in alignment with the measuring port (20),
- wherein the sleeve (16) including the test strip adapter (18) is arranged as a sealing barrier between the test strip (12) and the measuring port (20) in the protected state,
- **characterized in that** the test strip adapter (18) has an optical window (30) to establish a sealed optical measuring path between the test strip (12) and the measuring port (20) of the device (10).

2. Protective covering according to claim 1, wherein the test strip (12) can be inserted into the test strip adapter (18) by a user, wherein the test strip adapter (18) has holding elements (40) to engage and hold the test strip (12) in a defined position relative to the measuring port (20).

3. Protective covering according to claim 1 or 2, wherein the test strip adapter (18) can be detachably connected to the medical device (10) (10) via guiding or latching or plug-in elements (34).

4. Protective covering according to one of the claims 1 to 3, wherein the optical window (30) consists of a transparent material, preferably PET, PMMA, PC, PC-ABS, PS, SAN or ASA.

5. Protective covering according to one of the claims 1 to 4, further comprising a closure (42) to close the reception opening (32) for the device (10) when the sleeve (16) is slipped over the device (10).

6. Protective covering according to claim 5, wherein the sleeve (16) has a first closed end, and where the closure (42) provides a second closed end, such that the device (10) is fully protected against the environment.

7. Protective covering according to one of the claims 1 to 6, wherein the sleeve (16) is construed as a flexible unshaped member which can be fitted to the shape of the device (10).

8. Protective covering according to one of the claims 1 to 7, wherein the sleeve (16) consists of a flexible foil material which can be elastically adapted to an outer contour of the device (10).

9. Protective covering according to one of the claims 1 to 8, wherein the sleeve (16) is transparent at least in an area covering a display (28) or a control unit of the device (10).

10. Protective covering according to one of the claims 1 to 9, wherein the sleeve (16) comprises first and second sleeve parts (16',16") which can be connected by adherence in an overlapping joint region (38), such that the device (10) is fully enclosed in the combined sleeve (16).

11. Protective covering according to claim 10, wherein each of the sleeve parts (16',16") has a hermetically sealed closed end.

12. Protective covering according to one of the claims 1 to 11, wherein the sleeve (16) comprises a sack-like part which can be turned inside-out over test strip adapter (18).

13. Protective covering according to one of the claims 1 to 12, wherein the sleeve (16) comprises tear-off parts in a multi-layered arrangement which can be successively detached from the device (10) for sequential use.

14. Protective covering according to one of the claims 1 to 13, wherein the sleeve (16) is sterile and forms an aseptic shielding for the device (10).

15. Protective covering according to one of the claims 1 to 14, wherein the test strip adapter (18) is adapted to provide an automatic switch-on function of the device (10) when a test strip (12) is inserted.

## Patentansprüche

1. Schutzhülle für ein tragbares medizinisches Gerät (10), welches eine Messschnittstelle (20) für einen mit einer Probe beaufschlagbaren disposiblen analytischen Teststreifen (12) aufweist, umfassend
- eine vorzugsweise mit einer Aufnahmeöffnung (32) versehene Stulpe (16), die an dem Gerät (10) angebracht werden kann, um das Gerät (10) teilweise oder vollständig in einem betriebsfähigen geschützten Zustand einzuschließen,
- einen Teststreifenadapter (18), der als integraler Teil der Stulpe (16) ausgebildet ist, um einen Teststreifen (12) in Ausrichtung mit der Messschnittstelle (20) aufzunehmen,
- bei welchem die Stulpe (16) einschließlich des Teststreifenadapters (18) als eine Abdichtbarriere zwischen dem Teststreifen (12) und der Messschnittstelle (20) in dem geschützten Zustand angeordnet ist,
- **dadurch gekennzeichnet, dass** der Teststreifenadapter (18) ein optisches Fenster (30) aufweist, um einen abgedichteten optischen Messpfad zwischen dem Teststreifen (12) und der Messschnittstelle (20) des Geräts (10) bereitzustellen.

2. Schutzhülle nach Anspruch 1, bei welcher der Testreifen (12) durch einen Benutzer in den Teststreifenadapter (18) einsetzbar ist, wobei der Teststreifenadapter (18) Halteelemente (40) zum Eingreifen und Halten des Teststreifens (12) in einer definierten Position relativ zu der Messschnittstelle (20) aufweist.

3. Schutzhülle nach Anspruch 1 oder 2, bei welche der Teststreifenadapter (18) über Führungs- oder Rast- oder Steckelemente (34) lösbar mit dem medizinischen Gerät (10) verbindbar ist.

4. Schutzhülle nach einem der Ansprüche 1 bis 3, bei welcher das optische Fenster (30) aus einem transparenten Material besteht, vorzugsweise aus PET, PMMA, PC, PC-ABS, PS, SAN oder ASA.

5. Schutzhülle nach einem der Ansprüche 1 bis 4, weiterhin umfassend ein Schließmittel (42) zum Schließen der Aufnahmeöffnung (32) für das Gerät (10) wenn die Stulpe (16) über das Gerät (10) gestülpt ist.

6. Schutzhülle nach Anspruch 5, bei welcher die Stulpe (16) ein erstes geschlossenes Ende aufweist, und bei welcher das Schließmittel (42) ein zweites geschlossenes Ende bereitstellt, so dass das Gerät (10) vollständig gegenüber der Umgebung geschützt ist.

7. Schutzhülle nach einem der Ansprüche 1 bis 6, bei welcher die Stulpe (16) als flexibles ungeformtes Teil ausgebildet ist, welches an die Form des Geräts (10) anpassbar ist.

8. Schutzhülle nach einem der Ansprüche 1 bis 7, bei welcher die Stulpe (16) aus einem flexiblen Folienmaterial besteht, welches an eine äußere Kontur des Geräts (10) elastisch anpassbar ist.

9. Schutzhülle nach einem der Ansprüche 1 bis 8, bei welcher die Stulpe (16) zumindest in einem ein Display (28) oder eine Steuereinheit des Geräts (10) überdeckenden Bereich transparent ist.

10. Schutzhülle nach einem der Ansprüche 1 bis 9, bei welcher die Stulpe (16) erste und zweite Stulpenteile (16',16") umfasst, welche durch Klebekraft in einem überlappenden Verbindungsbereich (38) verbindbar sind, so dass das Gerät (10) in der zusammengeschlassenen Stulpe (16) vollständig eingeschlossen ist.

11. Schutzhülle nach Anspruch 10, bei welcher jedes der Stulpenteile (16',16") ein hermetisch abgedichtetes geschlossenes Ende aufweist.

12. Schutzhülle nach einem der Ansprüche 1 bis 11, bei welcher die Stulpe (16) einen sackförmigen Teil umfasst, der über den Teststreifenadapter (18) umstülpbar ist.

13. Schutzhülle nach einem der Ansprüche 1 bis 12, bei welcher die Stulpe (16) in mehrlagiger Anordnung Abreissteile umfasst, welche zum aufeinanderfolgenden Gebrauch nacheinander von dem Gerät (10) ablösbar sind.

14. Schutzhülle nach einem der Ansprüche 1 bis 13, bei welcher die Stulpe (16) steril ist und ein Sterilschild für das Gerät (10) bildet.

15. Schutzhülle nach einem der Ansprüche 1 bis 14, bei welcher der Teststreifenadapter (18) dazu ausgebildet ist, beim Einführen eines Teststreifens (12) eine automatische Einschaltfunktion für das Gerät (10) bereitzustellen.

## Revendications

1. Habillage de protection pour un dispositif (10) médical à main qui a un orifice (20) de mesure pour une bandelette de test (12) analytique jetable à laquelle un échantillon peut être appliqué comprenant
- un fourreau (16) qui préférablement a une ouverture (32) de réception et peut être monté sur le dispositif (10) pour renfermer partiellement ou totalement le dispositif (10) dans un état protégé utilisable,
- un adaptateur (18) de bandelette de test formé comme une partie intégrante du fourreau (16) pour recevoir une bandelette de test (12) en alignment avec l'orifice (20) de mesure,
- dans lequel le fourreau (16) incluant l'adaptateur (18) de bandelette de test est agencé comme une barrière de scellage entre la bandelette de test (12) et l'orifice (20) de mesure dans l'état protégé,
- **caractérisé en ce que** l'adaptateur (18) de bandelette de test a une fenêtre optique (30) pour établir un chemin optique de mesure scellé entre la bandelette de test (12) et l'orifice (20) de mesure du dispositif (10).

2. Habillage de protection selon la revendication 1, dans lequel la bandelette de test (12) peut être insérée dans l'adaptateur (18) de bandelette de test par un utilisateur, dans lequel l'adaptateur (18) de bandelette de test a des éléments (40) de maintien pour engager et maintenir la bandelette de test (12) dans une position définie par rapport à l'orifice (20) de mesure.

3. Habillage de protection selon la revendication 1 ou 2, dans lequel l'adaptateur (18) de bandelette de test peut être connecté de manière détachable au dispositif (10) médical par l'intermédiaire d'éléments (34) de guidage ou de verrouillage ou d'enfichage.

4. Habillage de protection selon l'une des revendications 1 à 3, dans lequel la fenêtre optique (30) consiste en un matériau transparent, préférablement PET, PMMA, PC, PC-ABS, PS, SAN ou ASA.

5. Habillage de protection selon l'une des revendications 1 à 4, comprenant en outre une fermeture (42) pour fermer l'ouverture (32) de réception pour le dispositif (10) lorsque le fourreau (16) est glissé par-dessus le dispositif (10).

6. Habillage de protection selon la revendication 5, dans lequel le fourreau (16) a une première extrémité fermée, et où la fermeture (42) offre une deuxième extrémité fermée, de telle sorte que le dispositif (10) est totalement protégé contre l'environnement.

7. Habillage de protection selon l'une des revendications 1 à 6, dans lequel le fourreau (16) est construit comme un élément flexible sans forme qui peut être ajusté à la forme du dispositif (10).

8. Habillage de protection selon l'une des revendications 1 à 7, dans lequel le fourreau (16) consiste en un matériau flexible en feuille qui peut être élastiquement adapté à un contour extérieur du dispositif (10).

9. Habillage de protection selon l'une des revendications 1 à 8, dans lequel le fourreau (16) est transparent au moins dans une zone recouvrant un afficheur (28) ou une unité de commande du dispositif (10).

10. Habillage de protection selon l'une des revendications 1 à 9, dans lequel le fourreau (16) comprend des première et deuxième parties (16', 16") de fourreau qui peuvent être connectées par adhérence dans une région de jonction (38) en chevauchement, de telle sorte que le dispositif (10) est totalement renfermé dans le fourreau (16) combiné.

11. Habillage de protection selon la revendication 10, dans lequel chacune des parties (16', 16") de fourreau a une extrémité fermée hermétiquement scellée.

12. Habillage de protection selon l'une des revendications 1 à 11, dans lequel le fourreau (16) comprend une partie de type sac qui peut être retournée par-dessus l'adaptateur (18) de bandelette de test.

13. Habillage de protection selon l'une des revendications 1 à 12, dans lequel le fourreau (16) comprend des parties déchirables dans un agencement à couches multiples qui peuvent être successivement détachées du dispositif (10) pour une utilisation séquentielle.

14. Habillage de protection selon l'une des revendications 1 à 13, dans lequel le fourreau (16) est stérile et forme une protection aseptique pour le dispositif (10).

15. Habillage de protection selon l'une des revendications 1 à 14, dans lequel l'adaptateur (18) de bandelette de test est adapté à offrir une fonction automatique d'activation du dispositif (10) lorsqu'une bandelette de test (12) est insérée.
